# EUROPEAN PATENT SPECIFICATION

(11) **EP 0 776 016 B1**
(45) Date of publication and mention of the grant of the patent: **03.03.2004**
(21) Application number: 96107298.0
(22) Date of filing: 08.05.1996
(51) Int. Cl.: H01G 4/35

(54) **Feedthrough capacitor assembly**
Zusammenbau von Durchführungskondensatoren
Assemblage de condensateurs de traversée

(30) Priority: 27.11.1995 US 7581
(43) Date of publication of application: 28.05.1997
(73) Proprietor: Greatbatch-Sierra, Inc., Carson City, Nevada 89706 (US)
(72) Inventor: Stevenson, Robert A., Canyon Country ,California 91351 (US); Ni, Richard H., Carson City, Nevada 89706 (US)
(74) Representative: Gerbaulet, Hannes, Dipl.-Ing.

(56) References cited:
- US-A- 5 333 095

## Description

This invention relates generally to improvements in ceramic feedthrough capacitors of the type used in implantable medical devices such as heart pacemakers and the like to decouple undesired interference signals from the device. The leads attaching the implanted device to the heart can act as antennae in the presence of a radiated EMI (electromagnetic interference) field (such as may be generated by cellular telephones).

More particularly, this invention relates to improved ceramic capacitor three terminal electrode plate configurations providing low impedance decoupling for EMI (electromagnetic interference) suppression, in combination with a common ground point through a separate coupling capacitor for electrical isolation of the filter circuit from the metal case of the medical device. The invention is particularly suited for use in defibrillators (tachycardia), heart pacemakers (bradycardia),and combined pacemaker defibrillator devices. Ceramic capacitors are appropriate for this application because of their high unit capacitance provided in a small size with a robust construction.

Ceramic capacitors are typically constructed by interleaving non-conductive layers of high dielectric constant ceramic material with metallic electrodes. The metallic electrodes are typically "laid-down" on the green ceramic material by silk screening processes. The device is then fired (sintered) to form a rigid monolithic structure (the "capacitor").

Well known in the art are feedthrough type monolithic ceramic capacitors, which offer a form factor which provides superior RF (radio frequency) performance for EMI filtering purposes. Reference U.S. Patent Nos. 4,148,003; 4,152,540; and 3,235,939.

Ceramic feedthrough capacitors have been adapted for EMI filtering of human implant devices (see U.S. Pat. No. 5,333,095). In order to provide proper EMI suppression, it is important that the feedthrough capacitor be directly bonded to the implantable device shield (titanium shell) or hermetic seal. This intimate relationship is essential so that the feedthrough capacitor acts as an integral part of the EMI shield so that undesirable EMI signals are reflected, or absorbed and decoupled before they can enter the interior of the device (inside the titanium shell) where they would disrupt the proper function of sensitive internal electronic circuits.

### SUMMARY OF THE INVENTION

In accordance with the features of claim 1, the feedthrough capacitor design incorporates novel electrode configurations which allow the internal filtering capacitor elements to be isolated. This allows for flexibility in implantable device (or other applications) waveform management. The preferred installation method for these novel electrode configuration feedthrough capacitors is in accordance with U.S. Patent Nos. 5,333,095 or 4,424,551. For human implant applications, the feedthrough lead wires are typically platinum, niobium or tantalum for resistance to body fluids. The hermetic terminal to which the feedthrough capacitor is mounted is typically made of titanium-alumina construction with gold brazing or glass sealing for resistance to body fluids.

Advantages of the novel capacitor matrix include:
A. Superior common and differential mode EMI suppression when compared to arrays of rectangular chip capacitors. Feedthrough technology offers greatly improved EMI suppression due to its transmission line type performance (low inductance, high resonant dip, ability to perform above resonance, i,e., broadband performance). Rectangular chip capacitors tend to resonate at a relatively low frequency, and, above resonance they become inductive and cease to function as an effective EMI filter.
B. Fewer components and high volumetric efficiency when compared to an array of monolithic chip capacitors (one feedthrough capacitor with novel electrodes according to the present invention replaces many individual chip capacitors).
C. The implantable defibrillator output waveform may be programmed for alternative circuits while retaining EMI suppression (further described herein).
D. The ability to decouple and suppress EMI right at the terminal-shield interface. This is an important feature when compared to MLC (monolithic capacitor) arrays, particularly when said chips are mounted at a distance from the shield can or housing (titanium). It is really too late to suppress EMI once it is inside of the implantable device housing, usually a shielding titanium case or can. The EMI is often characterized by a very short wavelength which is capable of re-radiating inside the can. The feedthrough type design of the invention actually suppresses the EMI before it enters the can interior where sensitive electronic circuits may be affected.

### DESCRIPTION OF THE DRAWINGS

FIGURE 1 is an isometric view of a bipolar feedthrough device with two capacitors C1 and C2 shown connected in series with a third capacitor C3, and depicting an isolated ground point (or common test point) G1;
FIGURE 2 is a schematic circuit diagram corresponding to the feedthrough device of FIG. 1;
FIGURES 3 and 4 are horizontal sectional views taken generally on the lines 3-3 and 4-4 of FIG. 1;
FIGURE 5 is a fragmented perspective view of the feedthrough device of FIG. 1, and depicting internal construction details thereof;
FIGURE 6 is a perspective view showing assembly of the feedthrough device of FIG. 1 with a terminal plate adapted for assembly with the housing or can of an implantable medical device;
FIGURE 7 is an isometric view of an alternative bipolar feedthrough device of discoidal shape, constructed according to the schematic circuit diagram of FIG. 2;
FIGURES 8 and 9 are a horizontal sectional views taken generally on the lines 8-8 and 9-9 of FIG. 7;
FIGURE 10 a perspective view showing assembly of the feedthrough device of FIG. 7 with a terminal plate adapted for assembly with the housing or can of an implantable medical device;
FIGURE 11 is an isometric view of another alternative bipolar feedthrough device, constructed according to the schematic circuit diagram of FIG. 2, with a central aperture for connection of the defibrillator pulse generator circuit (not shown) to the isolated ground point G1;
FIGURES 12 and 13 are horizontal sectional views taken generally on the lines 12-12 and 13-13 of FIG. 11;
FIGURE 14 is a perspective view showing assembly of the feedthrough device of FIG. 11 with a terminal plate adapted for assembly with the housing or can of an implantable medical device;
FIGURE 15 is an isometric view of a rectangular quad feedthrough device with a common isolated ground point G1;
FIGURE 16 is a schematic circuit diagram corresponding to the feedthrough device of FIG. 15;
FIGURES 17 and 18 are horizontal sectional views taken generally on the lines 17-17 and 18-18 of FIG. 15;
FIGURE 19 is an isometric view of a rectangular quad feedthrough device with isolated termination points G1 and G3;
FIGURE 20 is a schematic circuit diagram corresponding to the feedthrough device of FIG. 19;
FIGURES 21 and 22 are horizontal sectional views taken generally on the lines 21-21 and 22-22 of FIG. 19;
FIGURE 23 is an isometric view of a rectangular quad feedthrough device generally according to FIG. 19, but showing an alternative geometry with isolated termination points G1 and G3;
FIGURES 24 and 25 are horizontal sectional views taken generally on the lines 24-24 and 25-25 of FIG. 23;
FIGURE 26 is a fragmented perspective view of a quad feedthrough device according to FIG. 23, but showing the device in discoidal form;
FIGURE 27 is a perspective view showing assembly of the feedthrough device of FIG. 26 with a terminal plate adapted for assembly with the housing or can of an implantable medical device; and
FIGURE 28 is a perspective view showing assembly of a feedthrough device generally according to FIG. 19 but in discoidal form with a terminal plate adapted from assembly with the housing or can of an implantable medical device.

### DETAILED DESCRIPTION OF THE PREFERRED EMBODIMENTS

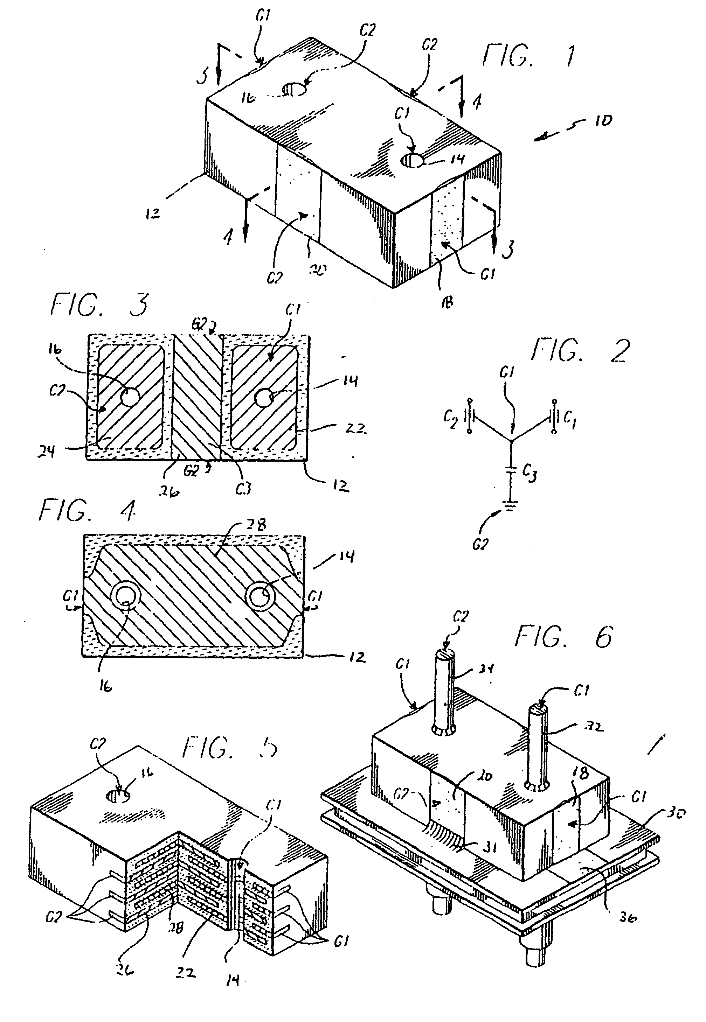

A rectangular bipolar feedthrough capacitor constructed in accordance with the present invention is shown in FIGURES 1 and 3-5. The feedthrough capacitor 10 comprises a multilayered construction having metallic conductive electrode plates arranged in alternating layers of different geometry and separated by dielectric material layers of ceramic or the like. The built-up assembly of multiple ceramic layers with thin metallic electrode plates applied thereto as by silk screening is generally known in the art and will not be discussed in further detail herein. The finished capacitor 10 is depicted in FIG. 1 in the form of a monolithic ceramic structure 12 having the metallic electrode plates embedded therein.

FIGURE 1 illustrates the rectangular feedthrough capacitor 10 having a pair of through ports 14 and 16 for receiving a corresponding pair of conductive terminal pins (not shown in FIG. 1). The surfaces of the through ports 14 and 16 are lined by surface metallization, as is known in the art, to connect the plates in parallel and facilitate conductive assembly with the terminal pins. In accordance with the invention, the through port 14 and the surface metallization thereof is conductively associated with a plurality of capacitor electrode plates associated with a first capacitor C1, while the second through port 16 and the surface metallization thereof is associated with a plurality of capacitor electrode plates associated with a second capacitor C2. The two capacitors C1 and C2 are in turn associated with a third capacitor C3 (not shown in FIG. 1) disposed electrically between a pair of electrical connection points G1 and G2 that are exposed by surface metallization 18 and 20, respectively, on discrete edge faces of the ceramic structure 12. FIGURE 2 shows an electrical schematic diagram conforming to the bipolar feedthrough capacitor shown in FIG. 1, with the capacitor C3 connecting the two feedthrough capacitors C1 and C2 to a common isolated ground point. It will be understood that the ground point defined at G2 does not represent the ground point of the pulse generator circuit of the medical device, but instead represents the metal housing or shield to which EMI signals are decoupled.

FIGURES 3 and 4 are sectional views illustrating alternate layers of metallic conductive electrode plates embedded within the structure of the ceramic body 12. FIGURE 3 shows a pair of electrode plates 22 and 24 associated respectively with the through ports 14 and 16, and in electrical isolation with a central plate 26 conforming to the ground point G2. FIGURE 4 shows an electrode plate 28 disposed in vertically stacked or sandwiched relation with the electrode plates of FIG. 3, and in electrical isolation to the through ports 14 and 16. When the capacitor 10 shown in FIG. 1 is assembled from a laminated stack-up of alternating plate layers as depicted in FIGS. 3 and 4, and as also depicted in partial section in FIG. 5, the bipolar feedthrough capacitor according to the circuit schematic of FIG. 2 is the result. It will be understood that a typical feedthrough capacitor 10 will incorporate a selected and typically large number of the conductive plate layers shown in FIGS. 3 and 4, in an alternating stack sequence.

FIGURE 6 shows the feedthrough capacitor 10 of FIGS. 1-5 assembled onto the inboard side of a terminal plate 30 adapted for hermetic and conductive mounting onto the housing of an implantable medical device or the like, so as to position the capacitor 10 within the interior of the housing for the medical device. In the preferred form, the G2 test point or points are designed to be grounded/bonded directly to the terminal plate 30, typically by use of a conductive thermoset material 31 such as conductive polyimide (e.q., Ablestik™ 71-1). This conductive thermoset material bonds to the G2 capacitor termination surface (typically a palladium-silver glass fired frit) and to the terminal plate 30 as shown. Terminal pins 32 and 34 can be electrically connected to the capacitors C1 and C2 respectively, by means of soldering or alternately by means of the conductive thermoset material. The G1 isolated ground point is desirably insulated from the conductive terminal plate 30 by a suitable insulation sheet 36 or the like. The isolated ground point G1 is accessible for facilitated electrical connection to other circuit components of the medical device or for electrical testing.

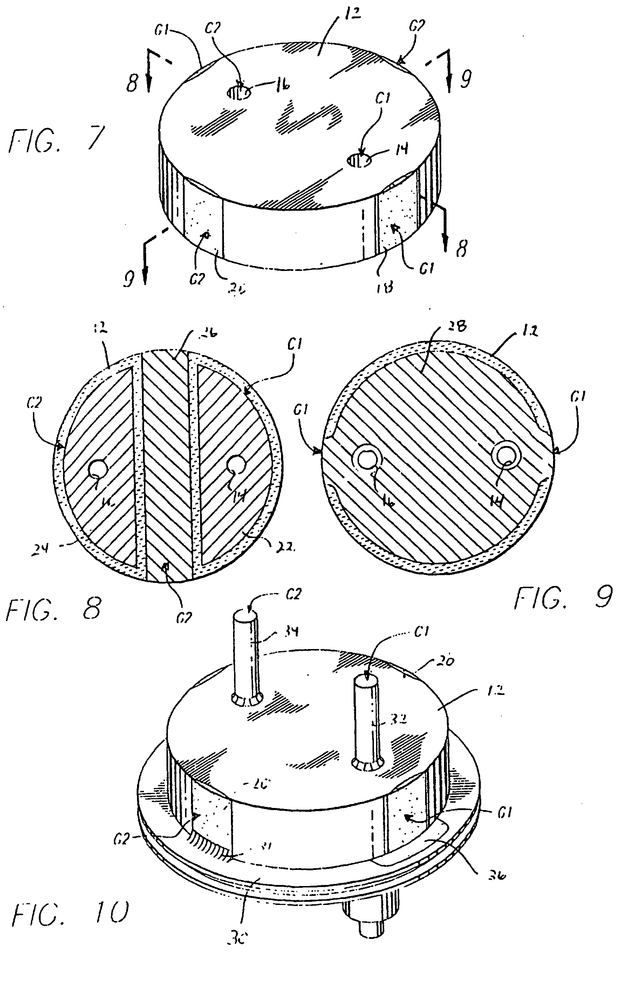

FIGURES 7-10 illustrate an alternative preferred form of the invention, wherein structural components conforming to those described in FIGS. 1 and 3-6 are identified by common reference numerals. The embodiment of FIGS. 7-10 differs only in the configuration of the bipolar feedthrough capacitor in a discoidal as opposed to a rectangular geometry.

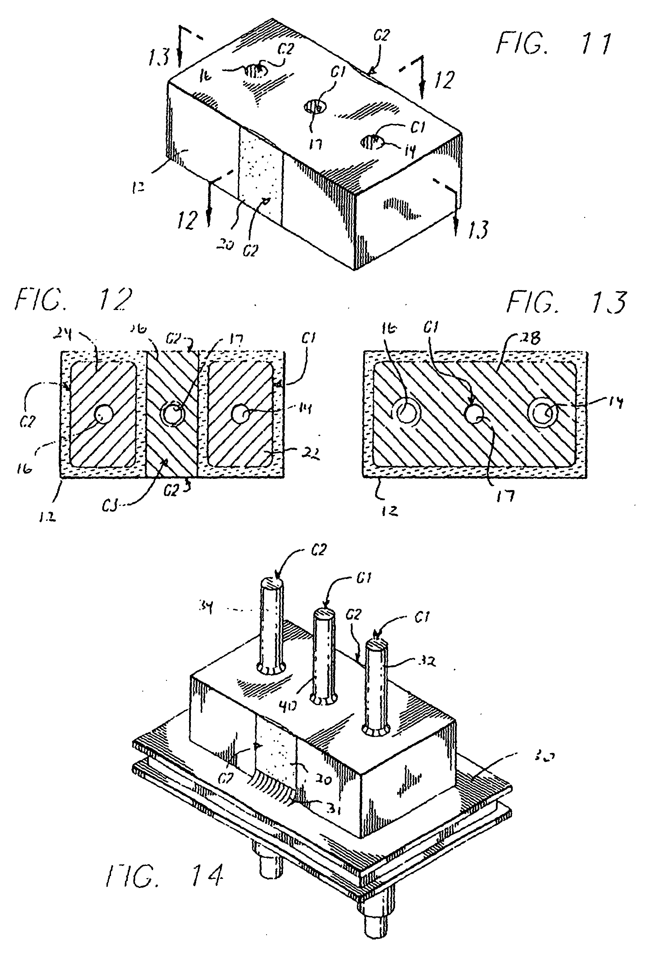

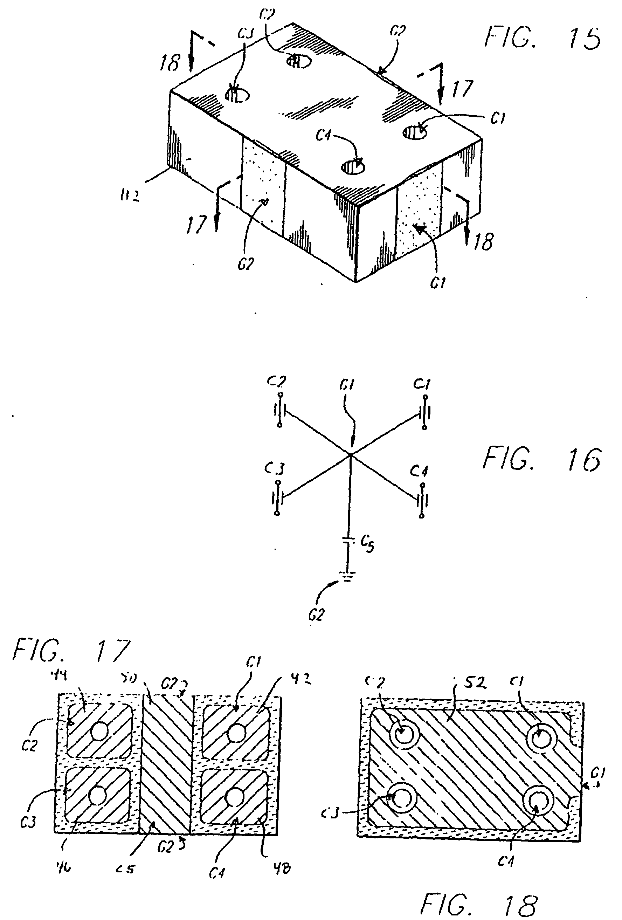

FIGURES 11-14 depict a further alternative preferred form of the invention, wherein components conforming with FIGS. 1 and 3-6 are also identified by the same reference numerals. FIGURES 11-14 show another bipolar feedthrough capacitor, having an alternative rectangular geometry wherein the isolated ground or test point G1 is defined by surface metallization applied to a third through port 17 extending through the capacitor structure 12 at a location between the C1 and C2 capacitor through ports 14 and 16, respectively. As shown in FIG. 14, electrical connection to the isolated ground or test point G1 is accomplished quickly and easily by electrically connecting a third terminal pin 40 thereto. In this embodiment, there is no requirement for additional insulating sheet elements for electrically isolating the test point G1 from the conductive terminal plate 30. Pin 40 typically does not extend all the way through to the body fluid side of the plate 30.

FIGURES 15-18 show a quad-type feedthrough capacitor constructed according to the principles shown and described with respect to FIGS. 1-14. As shown, a multilayer ceramic body 112 has alternating embedded layers of metallic electrode plates in a geometry to provide four separate capacitors C1, C2, C3, and C4 with isolated ground point G1 and ground point G2 including surface metallization on opposite sides of a fifth capacitor C5. This quad feedthrough capacitor is depicted by the circuit schematic of FIG. 16. FIG. 17 shows one layer geometry having active electrode plates 42, 44, 46, and 48 associated respectively with the capacitors C1, C2, C3, and C4, wherein these electrode plates are associated with individual terminal pin through ports lined with surface metallization as previously described. These active electrode plates are arranged in pairs, on opposite sides of a central plate 50 associated with the termination point G2. FIGURE 18 shows the electrode plate 52 associated with the test point G1, and formed in electrical isolation with the capacitor terminal pin through ports.

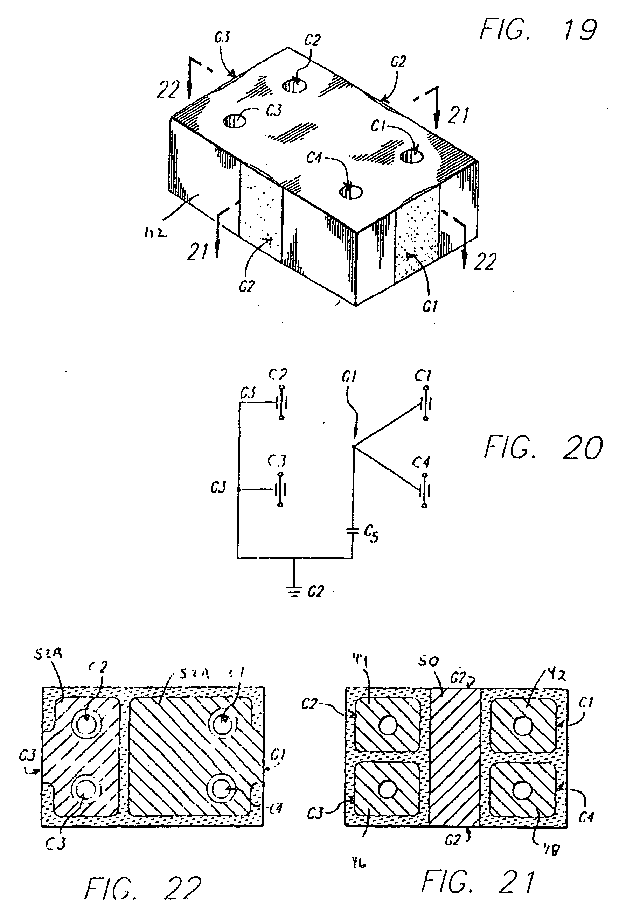

FIGURES 19-22 illustrate an alternative quad feedthrough capacitor with an additional ground point G3 isolated electrically from the isolated ground or test point G1. This alternative feedthrough capacitor is shown in electrical schematic form in FIG. 20 (various alternate schematic configurations are made possible by variations in the printed electrode patterns). Components previously shown and described with respect to FIG. 15-18 are identified by common reference numerals. The embodiment of FIGS. 19-22 differs only in that the G1 electrode plate 52 is subdivided into a pair of electrode subplates 52A and 52B associated respectively with the electrical connection points G1 and G3. This configuration allows the sense leads (lead wires which monitor the electrical activity of the heart) to be directly decoupled via C2 and C3 (for EMI purposes) to the metallic housing (typically titanium) of the medical device. The sensing circuits are very sensitive and are inherently more prone to disturbance from electrical electromagnetic interference (EMI). The C1 and C4 lead wires may then be used to conduct the high voltage (HV) defibrillator pulse to the heart. Capacitors C1 and C4 are electrically isolated through capacitor C5 from the shield housing of the medical device. This allows the defibrillator pulse to be electrically oriented from lead to lead, from either lead to device housing (case ground), from either lead to G1, or combinations of the above. This output waveform flexibility allows for a wide range of therapeutic options and the restoration of sinus rhythm to the heart.

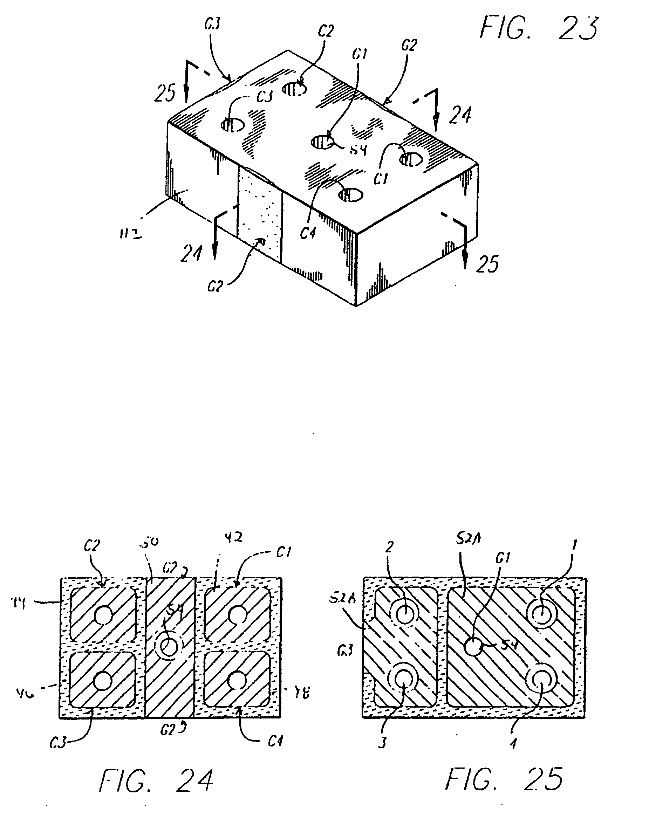

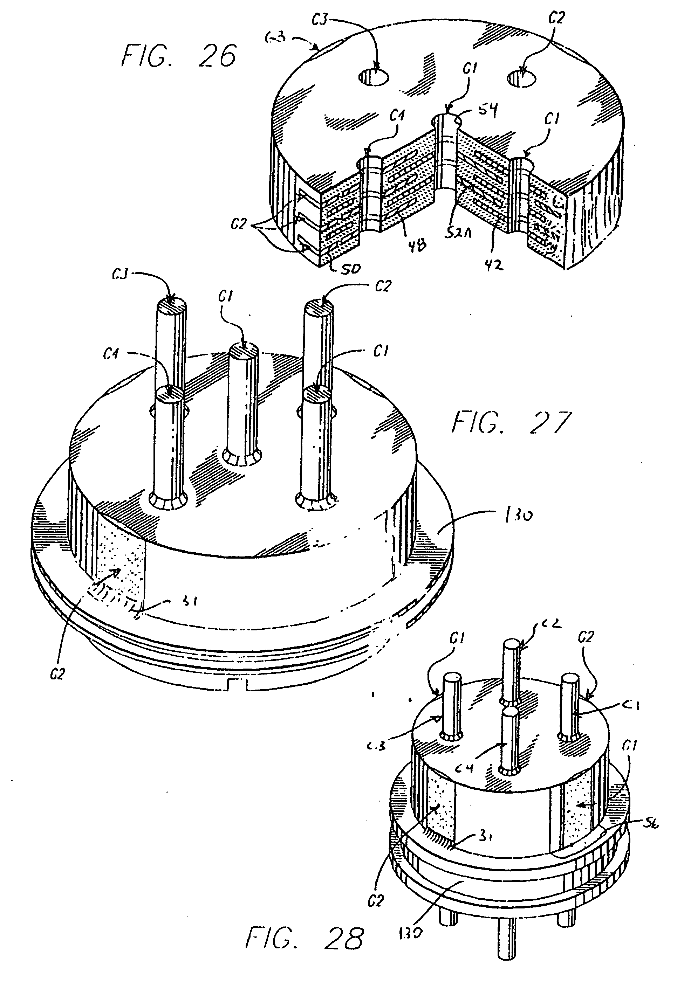

FIGURES 23-25 show a rectangular quad feedthrough capacitor conforming to FIGS. 19-22, except that the terminal point G1 is defined by surface metallization within a fifth through port 54. FIGURE 26 illustrates a quad-type feedthrough capacitor according to FIGS. 23-25, but in discoidal form. FIGURE 27 shows the discoidal quad capacitor of FIG. 26 mounted on a conductive terminal plate 130 for appropriate installation onto the typically titanium shield housing of the implantable medical device. Once again, the lead pin associated with the terminal point G1 typically does not extend to through the body fluid side of the assembled device.

FIGURE 28 shows an alternative configuration for a quad-type feedthrough capacitor of discoidal geometry, but wherein the terminal point G1 is exposed on the exterior edge of the discoidal capacitor, generally as described with respect to the rectangular quad capacitor of FIG. 19. In this geometry, insulation sheet 56 is required for electrically isolating the termination point G1 from the conductive base plate 130, as shown.

A variety of further modifications and variations to the invention will be apparent to those skilled in the art, such as additional capacitors and lead configurations and the like. For example, it will be understood that the active surface areas of the various conductive plates may be varied to permit selection of specific and proportioned capacitance values, such as variable selection of the active plate areas associated with the plates 22, 24 and 26 shown in FIG. 3. Accordingly, no limitation on the invention is intended by way of the foregoing description and accompanying drawings, except as set forth in the appended claims.

## Claims

1. A feedthrough capacitor assembly, comprising:
a substantially monolithic casing (12) of dielectric material having a plurality of conductive electrode plates (22, 24, 26, 28) encased therein in an alternating stack of a plurality of first and second layers in spaced parallel relation;
said first layer including at least one active plate (24, 22) corresponding to at least one feedthrough capacitor, and a first ground plate (26) electrically isolated from said active plate; and
said second layer including a second ground plate (28) common to said at least one of active plate of said first layer and cooperating therewith to define said feedthrough capacitor, and further cooperating with said first ground plate (26) to define a coupling capacitor for coupling said feedthrough capacitor to a common ground point (G2).

2. The feedthrough capacitor assembly of claim 1 wherein said first layer includes a plurality of electrically isolated active plates (24, 22, 26) corresponding respectively with a selected plurality of feedthrough capacitors (C1, C2, C3), said first ground plate (26) being electrically isolated from said active plates (24, 22), and said second ground plate (28) of said second layer cooperating with said plurality of active plates (24, 22), to define said plurality of feedthrough capacitors (C1, C2, C3).

3. The feedthrough capacitor assembly of claim 1 wherein said first layer includes a pair of said active plates (24, 26, 22) corresponding respectively to a pair of said feedthrough capacitors (C1, C2, C3).

4. The feedthrough capacitor assembly of claim 1 wherein said first layer includes at least four of said active plates (44, 42, 46, 48) corresponding respectively to at least four of said feedthrough capacitors (C1, C2, C3, C4).

5. The feedthrough capacitor assembly of claim 1 wherein said casing (12) is formed from a ceramic material.

6. The feedthrough capacitor assembly of claim 1 wherein said casing (12) has a rectangular shape.

7. The feedthrough capacitor assembly of claim 1 wherein said casing (12) has a discoidal shape.

8. The feedthrough capacitor assembly of claim 2 wherein said casing (12) has a plurality of through ports (14, 16) formed therein and disposed respectively to expose edges of said plurality of active plates (22, 24) of said first layer.

9. The feedthrough capacitor assembly of claim 8 wherein said through ports (14, 16) are lined with conductive material.

10. The feedthrough capacitor assembly of claim 9 further including a plurality of terminal pins (32, 34) mounted respectively within said through ports (14, 16).

11. The feedthrough capacitor assembly of claim 1 wherein said first ground plate (G2) of said first layer is exposed at one edge of said casing (12).

12. The feedthrough capacitor assembly of claim 11 wherein said second ground (G1) plate of said second layer is exposed at one edge of said casing (12).

13. The feedthrough capacitor assembly of claim 11 wherein said second ground plate (G1) of said second layer is exposed within a through port (17) formed in said casing (12).

14. The feedthrough capacitor assembly of claim 1 wherein said second ground plate (G1) of said second layer is exposed within a through port (17) formed in said casing (12).

15. The feedthrough capacitor assembly of claim 2 wherein said casing (12) has a plurality of through ports (14, 16, 17) formed therein and disposed respectively to expose edges of said active plates (22, 24, 26) of said first layer and said second ground plate (28) of said second layer, and first ground plate (26) of said first layer being electrically isolated from said through ports (14, 16, 17).

16. The feedthrough capacitor assembly of claim 1 further including a conductive terminal plate (30), said casing (12) being mounted on said terminal plate, and means for electrically connecting said first ground plate (G2) of said first layer to said terminal plate (30).

17. The feedthrough capacitor assembly of claim 16 further including means for isolating (36) said second ground (G1) plate of said second layer from said terminal plate (30).

18. The feedthrough capacitor assembly of claim 2 wherein said second ground plate (G1) of said second layer is subdivided into a pair of electrically isolated subplates, one of said subplates cooperating with a plurality of said active plates to define a plurality of said feedthrough capacitors and cooperating with said first ground plate (G2) to define said coupling capacitor (C1, C2, C3, C4) and the other of said subplates cooperating with at least one additional active plate (50) of said first layer to define at least one additional feedthrough capacitor (C5), and means for connecting said other of said subplates directly to said common ground port.

## Patentansprüche

1. Durchführungskondensatoranordnung, umfassend
ein im Wesentlichen einstückiges Gehäuse (12) aus dielektrischem Werkstoff, das mehrere leitende Elektrodenplatten (22, 24, 26, 28) in einem abwechselnden Stapel mehrerer erster und zweiter Lagen parallel zueinander mit Abstand voneinander aufnimmt,
wobei die genannte erste Lage mindestens eine aktive Platte (24, 22) enthält, die mindestens einem Durchführungskondensator entspricht, und eine erste Erdungsplatte (26), die von der genannten aktiven Platte elektrisch isoliert ist, und
wobei die genannte zweite Lage eine zweite Erdungsplatte (28) enthält, die der mindestens einen aktiven Platte der genannten ersten Lage gemeinsam ist und damit zusammenarbeitet, um den genannten Durchführungskondensator zu bilden, und weiterhin mit der ersten Erdungsplatte (26) zusammenarbeitet, um einen Koppelkondensator zu bilden, um den genannten Durchführungskondensator an einen gemeinsamen Erdungspunkt (G2) zu schalten.

2. Durchführungskondensatoranordnung nach Patentanspruch 1, in der die genannte erste Lage mehrere elektrisch isolierte aktive Platten (24, 22, 26) umfasst, die mehreren ausgewählten Durchführungskondensatoren (C1, C2, C3) entsprechen, wobei die genannte erste Erdungsplatte (26) von den genannten aktiven Platten (24, 22) elektrisch isoliert ist und die genannte zweite Erdungsplatte (28) der genannten zweiten Lage mit den genannten mehreren aktiven Platten (24, 22) zusammenarbeitet, um die genannten mehreren Durchführungskondensatoren (C1, C2, C3) zu bilden.

3. Durchführungskondensatoranordnung nach Patentanspruch 1, in der die genannte erste Lage ein Paar der genannten aktiven Platten (24, 26, 22) umfasst, die jeweils einem Paar der genannten Durchführungskondensatoren (C1, C2, C3) entsprechen.

4. Durchführungskondensatoranordnung nach Patentanspruch 1, in der die genannte erste Lage mindestens vier der genannten aktiven Platten (44, 42, 46, 48) umfasst, die jeweils mindestens vier der genannten Durchführungskondensatoren (C1, C2, C3, C4) entsprechen.

5. Durchführungskondensatoranordnung nach Patentanspruch 1, in der das genannte Gehäuse (12) aus einem keramischen Werkstoff geformt ist.

6. Durchführungskondensatoranordnung nach Patentanspruch 1, in der das genannte Gehäuse (12) eine rechtwinklige Form hat.

7. Durchführungskondensatoranordnung nach Patentanspruch 1, in der das genannte Gehäuse (12) eine scheibenartige Form hat.

8. Durchführungskondensatoranordnung nach Patentanspruch 2, in der das genannte Gehäuse (12) mehrere darin ausgebildete Durchgangsöffnungen (14, 16) aufweist, die jeweils so angeordnet sind, dass Kanten der genannten mehreren aktiven Platten (22, 24) der genannten ersten Lage offen liegen.

9. Durchführungskondensatoranordnung nach Patentanspruch 8, in der die genannten Durchgangsöffnungen (14, 16) mit leitfähigem Werkstoff ausgekleidet sind.

10. Durchführungskondensatoranordnung nach Patentanspruch 9, außerdem mehrere Anschlüsse (32, 34) enthaltend, die jeweils in den genannten Durchgangsöffnungen (14, 16) angebracht sind.

11. Durchführungskondensatoranordnung nach Patentanspruch 1, in der die genannte erste Erdungsplatte (G2) der genannten ersten Lage an einer Kante des genannten Gehäuses (12) offen liegt.

12. Durchführungskondensatoranordnung nach Patentanspruch 11, in der die genannte zweite Erdungsplatte (G1) der genannten zweiten Lage an einer Kante des genannten Gehäuses (12) offen liegt.

13. Durchführungskondensatoranordnung nach Patentanspruch 11, in der die genannte zweite Erdungsplatte (G1) der genannten zweiten Lage in einer Durchgangsöffnung (17) offen liegt, die in dem genannten Gehäuse (12) ausgebildet ist.

14. Durchführungskondensatoranordnung nach Patentanspruch 1, in der die genannte zweite Erdungsplatte (G1) der genannten zweiten Lage in einer Durchgangsöffnung (17) offen liegt, die in dem genannten Gehäuse (12) ausgebildet ist.

15. Durchführungskondensatoranordnung nach Patentanspruch 2, in der das genannte Gehäuse (12) mehrere darin ausgebildete Durchgangsöffnungen (14, 16, 17) aufweist, die jeweils so angeordnet sind, dass Kanten der genannten aktiven Platten (22, 24, 26) der genannten ersten Lage offen liegen und in der die genannte zweite Erdungsplatte (28) der genannten zweiten Lage und die erste Erdungsplatte (26) der genannten ersten Lage von den genannten Durchgangsöffnungen (14, 16, 17) elektrisch isoliert sind.

16. Durchführungskondensatoranordnung nach Patentanspruch 1, außerdem eine leitfähige Anschlussplatte (30) aufweisend, wobei das genannte Gehäuse (12) auf der genannten Anschlussplatte montiert ist, und Mittel zum elektrischen Anschluss der genannten ersten Erdungsplatte (G2) der genannten ersten Lage an die genannte Anschlussplatte (30).

17. Durchführungskondensatoranordnung nach Patentanspruch 16, außerdem Mittel zur Isolierung (36) der genannten zweiten Erdungsplatte (G1) der genannten zweiten Lage von der genannten Anschlussplatte (30) aufweisend.

18. Durchführungskondensatoranordnung nach Patentanspruch 2, in der die genannte zweite Erdungsplatte (G1) der genannten zweiten Lage in ein Paar elektrisch isolierter Unterplatten unterteilt ist, wobei eine der genannten Unterplatten mit mehreren der genannten aktiven Platten zusammenarbeitet, um mehrere der genannten Durchführungskondensatoren zu bilden, und mit der genannten ersten Erdungsplatte (G2) zusammenarbeitet, um den genannten Koppelkondensator (C1, C2, C3, C4) zu bilden, und wobei die andere der genannten Unterplatten mit mindestens einer zusätzlichen aktiven Platte (10) der genannten ersten Lage zusammenarbeitet, um mindestens einen zusätzlichen Durchführungskondensator (C5) zu bilden, und Mittel, um die genannte andere der genannten Unterplatten direkt an die gemeinsame Erdungsöffnung anzuschließen.

## Revendications

1. Montage de condensateur de passage comprenant :
un boîtier substantiellement monolithique (12) en matériau diélectrique ayant une pluralité de lames d'électrodes conductrices (22, 24, 26, 28) qui y sont encastrés en une pile alternée d'une pluralité de premières et de secondes couches espacées parallèlement ;
la première couche comprenant au moins une lame active (24, 28), correspondant à au moins un condensateur de passage, et une première plaque de terre (26) isolée électriquement de la lame active et
la seconde couche comprenant une seconde plaque de terre (28) commune à au moins une lame active de la première couche et coopérant avec celle-ci pour définir le condensateur de passage et coopérant de plus avec la première plaque de terre (26) pour définir un condensateur de couplage pour coupler le condensateur de passage à un point de terre commun (G2).

2. Montage de condensateur de passage selon la revendication 1 dans lequel la première couche comprend une pluralité de lames actives isolées électriquement (24, 22, 26) correspondant respectivement à une pluralité sélectionnée de condensateurs de passage (C1, C2, C3), la première plaque de terre (26) étant isolée électriquement des lames actives (24, 26) et la seconde plaque de terre (28) de la seconde couche coopérant avec la pluralité de lames actives (24 ,22) pour définir la pluralité de condensateurs de passage (C1, C2, C3).

3. Montage de condensateur de passage selon la revendication 1 dans lequel la première couche comprend une paire de lames actives (24, 26, 27) correspondant respectivement à une paire de condensateurs de passage (C1, C2, C3).

4. Montage de condensateur de passage selon la revendication 1 dans lequel la première couche comprend au moins quatre lames actives (44, 42, 46, 48) correspondant respectivement à au moins quatre condensateurs de passage (C1, C2, C3, C4).

5. Montage de condensateur de passage selon la revendication 1 dans lequel le boîtier (18) est formé en un matériau céramique.

6. Montage de condensateur de passage selon la revendication 1 dans lequel le boîtier (18) a une forme rectangulaire.

7. Montage de condensateur de passage selon la revendication 1 dans lequel le boîtier (18) a une forme en forme de disque.

8. Montage de condensateur de passage selon la revendication 2 dans lequel le boîtier (18) a une pluralité d'orifices de passage (14, 16) qui y sont formés et disposés respectivement pour dénuder les arêtes de la pluralité de lames actives (22, 24) de la première couche.

9. Montage de condensateur de passage selon la revendication 8 dans lequel les orifices de passage (14, 16) sont recouverts de matériau conducteur.

10. Montage de condensateur de passage selon la revendication 9 comprenant de plus une pluralité de bornes (32, 34) montées respectivement à l'intérieur des orifices de passage (14, 16).

11. Montage de condensateur de passage selon la revendication 1 dans lequel la première plaque de terre (G2) de la première couche est dénudée à une arête du boîtier (12).

12. Montage de condensateur de passage selon la revendication 11 dans lequel la seconde plaque de terre (G1) de la seconde couche est dénudée à une arête du boîtier (12).

13. Montage de condensateur de passage selon la revendication 11 dans lequel la seconde plaque de terre (G1) de la seconde couche est dénudée à l'intérieur d'un orifice de passage (17) formé dans le boîtier (12).

14. Montage de condensateur de passage selon la revendication 1 dans lequel la seconde plaque de terre (G1) de la seconde couche est dénudée à l'intérieur d'un orifice de passage (17) formé dans le boîtier (12).

15. Montage de condensateur de passage selon la revendication 2 dans lequel le boîtier (12) a une pluralité d'orifices de passage (14, 16, 17) qui y sont formés et disposés respectivement pour dénuder les arêtes des lames actives (22, 24, 26) de la première couche et de la seconde plaque de terre (28) de la seconde couche et la première plaque de terre (26) de la première couche étant isolée électriquement des orifices de passage (14, 16, 17).

16. Montage de condensateur de passage selon la revendication 1 comprenant de plus une plaque de raccordement (30), le boîtier (12) étant monté sur la plaque de raccordement, et des moyens pour connecter électriquement la première plaque de terre (G2) de la première couche à la plaque de raccordement (30).

17. Montage de condensateur de passage selon la revendication 16 comprenant de plus des moyens pour isoler (36) la seconde plaque de terre (G1) de la seconde couche de la plaque de raccordement (30).

18. Montage de condensateur de passage selon la revendication 2 dans lequel la seconde plaque de terre (G1) de la seconde couche est subdivisée en une paire de sous-plaques isolées électriquement, l'une des sous-plaques coopérant avec une pluralité de lames actives pour définir une pluralité de condensateurs de passage et coopérant avec la première plaque de terre (G1) pour définir le condensateur de couplage (C1, C2, C3, C4) et l'autre sous-plaque coopérant avec au moins une lame active supplémentaire (50) de la première couche pour définir au moins un condensateur de passage supplémentaire (C5) et des moyens pour relier l'autre sous-plaque directement à l'orifice de terre commun.
